# EUROPEAN PATENT APPLICATION

(11) **EP 0 667 127 A1**
(43) Date of publication of application: **16.08.1995**
(21) Application number: 95200317.6
(22) Date of filing: 09.02.1995
(51) Int. Cl.: A61B 17/58

(54) **Device for implantation for the purpose of limiting the movements between two vertebrae**

(30) Priority: 10.02.1994 NL 9400210
(71) Applicant: ACROMED B.V., NL-3034 KH Rotterdam (NL)
(72) Inventor: Sanders, Marcus Maria, NL-2411 BT Bodegraven (NL)
(74) Representative: Smulders, Theodorus A.H.J., Ir.

(57) **Abstract**

The invention relates to a device for implantation for the purpose of limiting movements between two vertebrae, comprising at least two vertebra engaging elements each having a free end, and an elastic coupling element which is connected with the free ends of the vertebra engaging elements, the elastic coupling element being designed in such a manner that upon an increase of the distance between the free ends of the vertebra engaging elements starting from a neutral position the coupling element generates a force counteracting the increase and upon a decrease of the distance between the free ends of the vertebra engaging elements starting from the neutral position the coupling element generates a force counteracting the decrease.

## Description

This invention relates to a device to be implanted for the purpose of limiting movements between two vertebrae.

Heretofore, there have been only two forms of treating back complaints in the lower part of the back caused by an unstable vertebral column segment. Characteristic of an unstable vertebral column segment is that a slight displacement from the neutral position results in too great a change or an abnormal change in the shape of the vertebral column segment. The complaints of pain are treated on the one hand with conservative methods such as physiotherapy and anti-inflammatory injections and on the other with relatively aggressive methods based on arthrodesis, i.e. fusion of the unstable vertebral elements. Between these two treatment options lies a wide gap which could not be filled heretofore. This gap could very suitably be filled by implantation of a device permitting limited movement between the vertebrae.

Movement-limiting devices for vertebrae could also be very suitably fitted at the transition between vertebral column segments fused by arthrodesis and the non-fused vertebral column parts adjacent to these segments. The vertebrae located at the transition between a fused and a non-fused vertebral column part are loaded extra heavily since these vertebrae attempt to take up the movements which were previously taken up by the fused vertebral column part. In order to relieve these vertebrae and the surrounding muscles to some extent, a movement-limiting device could very effectively be employed at precisely that point.

In the case of knee joints, for instance, it is known to use elastic tensioning bands which are tensioned around the free ends of two joint engaging elements, such as for instance screws, which are each fitted in a joint member whose relative movements are to be limited. Such a device is described in EP-A-0 260 970. The drawback of devices functioning on the basis of elastic tensioning bands is that the movements of the joint members are counteracted in only one direction by a force produced by the tensioning bands, viz. in that direction in which the band is pulled taut. Movements by which the tension on the elastic bands is reduced are, of course, not limited and even promoted. Since vertebrae can move relative to each other in all directions, the principle of limiting the movements by means of elastic bands cannot successfully be transferred to the vertebral column.

Another drawback of the known devices equipped with elastic tensioning bands is that the tensioning bands, even when the joint members are disposed in the neutral position relative to each other, are under stress and therefore draw the joint members toward each other. The element which is to produce the counterpressure to keep the joint members spaced apart is formed by a cartilage part or a connective tissue structure such as, for instance, a meniscus or, when the device is applied to the vertebral column, an intervertebral disk. This cartilage part loses its elasticity under the influence of the continuous load and moreover becomes thinner in the course of time. As a result, the tension on the tensioning bands lessens, so that the device allows greater movement again and the chances of the tensioning bands coming off the free ends of the screws increase.

Further, the durability of the tensioning bands, typically made of rubber or plastic, is not sufficient to function satisfactorily for the rest of the patient's life under the fatigue loads exerted thereon.

The object of the invention is to provide a device for limiting movements between two vertebrae without the above-described disadvantages.

To that end, the device comprises at least two vertebra engaging elements each having a free end, and an elastic coupling element which is connected with the free ends of the vertebra engaging elements, the elastic coupling element being designed in such a manner that upon an increase of the distance between the free ends of the vertebra engaging elements starting from a neutral position the coupling element generates a force counteracting the increase, and upon a decrease of the distance between the free ends of the vertebra engaging elements starting from the neutral position the coupling element generates a force counteracting the decrease.

Such a device has the advantage that also in the case where the free ends of the vertebra engaging elements move towards each other, the coupling element will produce a force counteracting this movement. This provides the advantage that in the event of such movements the intervertebral disk is loaded to a lesser extent. Moreover, the coupling element need not exert any force on the vertebra engaging elements when they are in the neutral position. This prevents an additional force being exerted continuously on the intervertebral disk, so that the extent of thinning of the intervertebral disk over a particular period of time will be slight.

According to a further elaboration of the invention, it is particularly advantageous if the force counteracting the increase or decrease of the distance between the free ends of the vertebra engaging elements increases progressively with the amount of increase or decrease. Such progressive increase of the force upon an increase or decrease of the distance between the free ends of the vertebra engaging elements provides that the movement ends in a damped manner against a kind of end stop. Thus shock loads to which the vertebrae are exposed are minimized.

In further elaboration of the invention, the device is characterized in that the counteractive force increases exponentially with the amount of increase or decrease of the distance between the free ends of the vertebra engaging elements.

Tests show that such exponential increase is highly satisfactory and that the occurrence of shock loads is thereby minimized.

The vertebra engaging elements will mainly be arranged on the dorsal side of the vertebral column. Accordingly, upon forward bending, also referred to as flexion, the distance between the free ends of the vertebra engaging elements increases while, conversely, upon straightening of the vertebral column, also referred to as extension, the distance between the free ends of the vertebra engaging elements decreases.

According to a further elaboration of the invention, it is particularly advantageous if the counteractive force arising upon a particular increase of the distance between the free ends of the vertebra engaging elements is smaller than the counteractive force arising upon an equally large decrease of that distance. The consequence of this is that unbending the vertebral column will require more effort than bending it, which corresponds with the natural situation in the case of a healthy vertebral column.

A first embodiment of the above-described device is characterized in that the coupling element comprises two flexible pins which are each rigidly connected by one end to the free end of a vertebra engaging element and are each connected at their other end to a rigid connecting member, each vertebra engaging element comprising at the free end thereof an internal cavity in which the pin extends at least partly, the internal surface of the cavity serving to support the pin during the bending thereof.

Such a device provides the advantage that all parts can be made of metal. Metal presents few problems in the natural environment in which the device is disposed. Rubber and plastic have a more limited resistance to fatigue than metal does, which is unfavorable in particular when they are employed as an elastic element. Accordingly, the first embodiment, in which no rubber or plastic is used, is particularly advantageous.

Preferably, the internal surface of the cavity in the vertebra engaging element is designed in such a manner that upon a greater amount of increase or decrease of the distance between the free ends of the vertebra engaging elements, i.e. upon a stronger bending of the pins, the pins abut against the internal surface over a greater part of their length.

Because upon the bending of the pin the pin abuts against the internal surface of the internal cavity over an increasingly greater part of its length, the force counteracting the bending increases progressively upon an increase of the bending. This is the result of the shortening of the flexible part of the pin. Through an appropriate design of the internal surface of the cavity in which the pin extends, it is, for instance, possible to obtain an exponential increase of the counteractive force upon an increase or decrease of the distance between the vertebra engaging elements starting from the neutral position thereof.

The flexible pin is preferably manufactured from memory metal, such as for instance a TiNi alloy. Such material provides the advantage that it can resist an elongation of approximately 8% in the elastic range. With a relatively short pin, which is necessary to limit the required overall space for the device, such an amount of elongation still enables a relatively large displacement of the free pin end relative to the clamped pin end.

In order to limit the undesirable build-up of moment in the memory metal pins and the rigid connecting member of the coupling element, it is particularly advantageous, in accordance with a further elaboration of the invention, when the rigid connecting member is pivotally connected to the free ends of the two flexible pins.

To limit the maximum elongation in the pins, it can be particularly advantageous, according to a further elaboration of the invention, if the pins are designed as a bundle of substantially parallel wire-shaped elements. Such bundle of wire-shaped elements can be bent to a considerable extent without the elongation in the individual wire-shaped elements becoming inadmissibly large. This provides that even in the case of extreme bending, the deformations still take place within the elastic range of the material.

A first alternative embodiment of the invention is characterized in that the coupling element comprises a rigid connecting member and two ball-and-socket joints, of which each ball in mounted condition is rigidly connected with an associated vertebra engaging element, while each socket is slidably connected with the rigid connecting member and in the neutral position is urged in an end position by biassed springing means.

Over the known device, this device has an important advantage in that both with flexion and with extension of the vertebral column, a force counteracting this flexion or extension is generated by the device.

In order to effect a progressive increase of the force counteracting the change in the distance between the free ends of the vertebra engaging elements, it is possible, in accordance with a further elaboration of the invention, for the springing means to be formed by blocks made from rubber or plastic and accommodated in a chamber of the rigid connecting member. As long as the rubber or plastic blocks can still deform freely, the counteractive force will increase approximately linearly with the change in the distance between the free ends of the vertebra engaging elements. However, as soon as the blocks contact the walls of the chamber, the force counteracting the change in the distance will increase progressively due to the fact that the rubber blocks can no longer expand freely in particular directions.

In this connection, according to a further elaboration of the invention, the two sockets can each be positioned relative to the springing means in such a manner that upon an increase of the distance between the free ends of the vertebra engaging elements one socket is urged into the associated end position and the other socket moves from the end position against the spring pressure of the springing means associated with that socket, while upon a decrease of the distance between the free ends of the vertebra engaging elements the other socket is urged into the end position and the one socket moves from the end position against the spring pressure of the springing means associated with that one socket. Thus, both for an increase and for a decrease of the distance between the vertebra engaging elements, in each case only one of the blocks made of rubber or plastic is operative. By choosing, for instance, blocks having different spring characteristics, in this way the counteractive force upon a particular decrease of the distance can simply be different from the counteractive force upon an equally large increase of the distance between the free ends of the vertebra engaging elements. It can thus be provided that for flexion, for instance, less resistance has to be overcome than for a similar extension, which corresponds with the natural situation in the case of a healthy vertebral column.

In a third embodiment according to the invention, the coupling element comprises at least one spring element, manufactured from springing wire or sheet material, which is provided with a curvature, the or each spring element being connected at the free end thereof with the free ends of the vertebra engaging elements. Such element, manufactured from springing wire or sheet material and provided with a curvature, upon an increase of the distance between the free ends of the vertebra engaging elements, exerts on the vertebra engaging elements a force counteracting the increase of the distance. According as the distance between the free ends of the vertebra engaging elements increases, the curvature in the spring element is straightened, so that the spring arm becomes smaller and the force needed to further extend the spring increases progressively.

In order to provide that the counteractive force increases progressively also in the case where the free ends of the vertebra engaging elements move toward each other, it is particularly advantageous, in accordance with a further elaboration of the invention, if the coupling element comprises two spring elements, the curvatures of the two elements being so designed that upon a decrease of the distance between the free ends of the vertebra engaging elements the spring elements butt against each other. Owing to the fact that the springs butt against each other upon a particular reduction of the distance between the free ends of the vertebra engaging elements, much more counteractive force will have to be overcome to achieve a further reduction of the distance. Through the interaction between the two spring elements, in particular in that the spring elements must bend over a shorter part of their length, the spring characteristic will change markedly. This change of the spring characteristic results in a progressive increase of the counteractive force upon the decrease of the distance between the free ends of the vertebra engaging elements.

When the spring elements are designed as leaf springs, the chances of the spring elements sliding past each other rather than butting against each other are minimized.

To clarify the invention two exemplary embodiments of the device are described with reference to the drawing.

Fig. 1 is a lateral view of a part of the lumbar part of the vertebral column in which a first exemplary embodiment of the device is fitted;
Fig. 2 is a cranial view taken on line II-II of Fig. 1;
Fig. 3 is a side elevation of a first embodiment of the device in the neutral position;
Fig. 4 is a side elevation of the device, similar to that shown in Fig. 3, where the distance between the free ends of the vertebra engaging elements is reduced;
Fig. 5 is a side elevation of a second embodiment of the device according to the invention;
Fig. 6 is a top plan view of a third embodiment of the device;
Fig. 7 is a top plan view of a fourth embodiment of the device; and
Fig. 8 is a side elevation of the device shown in Figs. 6 and 7.

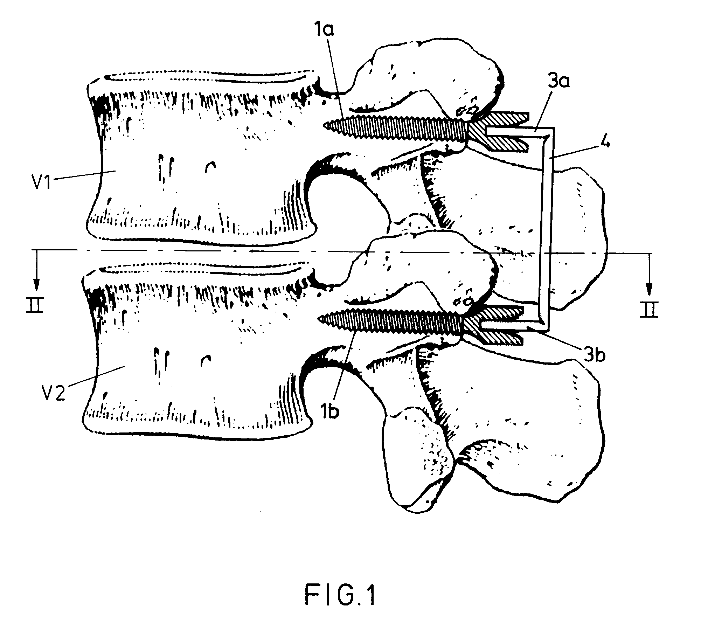

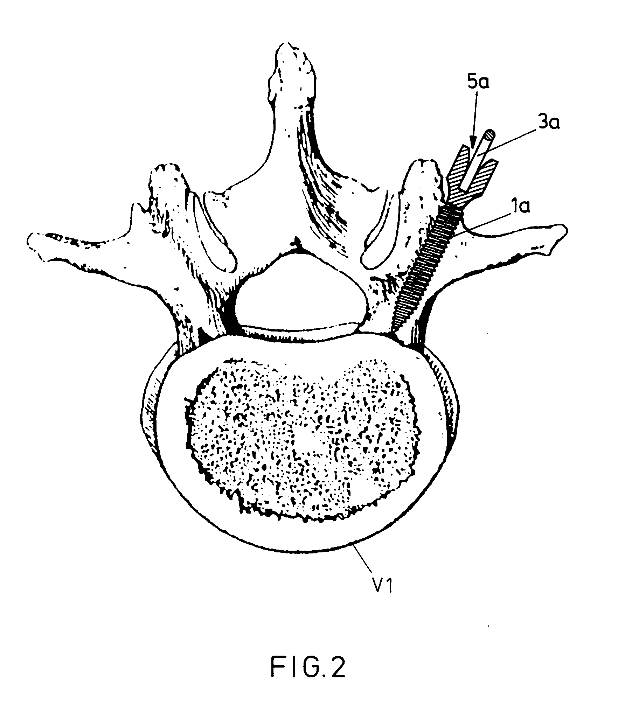

Fig. 1 shows the position of vertebra engaging elements 1 a, 1 b of the device according to the first exemplary embodiment, which is preferred, in two lumbar vertebrae V1, V2, located one above the other, while Fig. 2 shows the position of a vertebra engaging element 1 b in cranial view.

In the exemplary embodiments shown, the vertebra engaging elements 1 a, 1 are each designed as pedicle screws. It will be clear, however, that for the vertebra engaging elements other engagement constructions to be rigidly connected to the vertebrae can be used as well, such as for instance the transversal hook proposed in applicant's patent application EP-A-0 564 046.

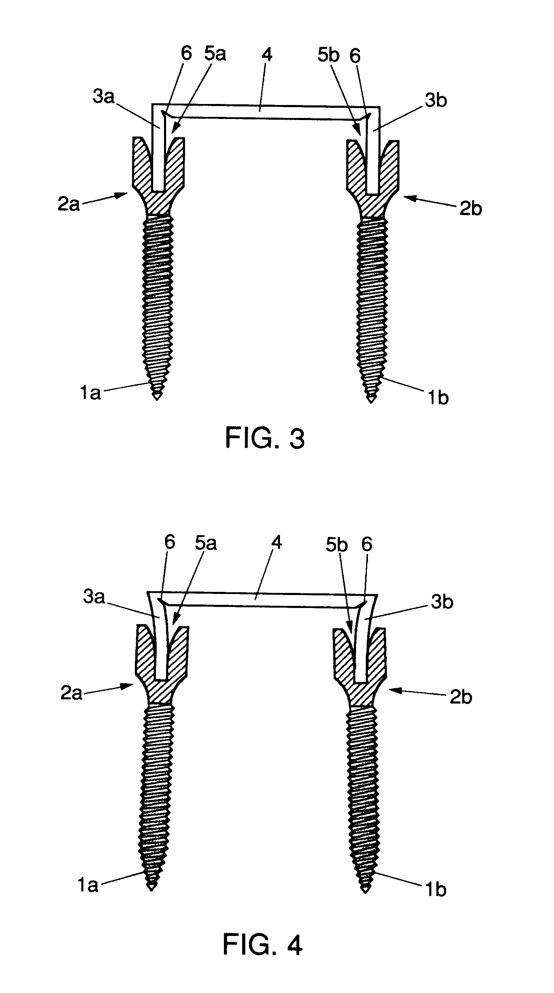

Figs. 3 and 4 respectively show the device attached to the vertebrae in Figs. 1 and 2 in the neutral position and in a position where the distance between the free ends 2a, 2b of the pedicle screws 1a, 1 b is reduced. When the vertebral column is in the natural position, the free ends 2a, 2b of the pedicle screws 1 a, 1 are spaced apart a specific distance. In this situation the pedicle screws 1 a, 1 are in the neutral position.

According to the invention, the device includes a coupling element which comprises two flexible pins 3a, 3b and a rigid connecting member 4. The flexible pins 3 are each rigidly connected by a first end to a pedicle screw 1 and connected by a second end to the rigid connecting member 4. The pedicle screws 1a, 1 are both provided, at the free end 2a, 2b thereof, with an internal cavity 5a, 5b in which the pin 3a, 3b extends at least over a part of its length. The internal surface of the cavity 5a, 5b is of such design that the pin 3a, 3b, upon being bent, abuts against and is supported by the internal surface of the cavity 5a, 5b. Preferably, the surface is so designed that upon a greater extent of increase or decrease of the distance between the free ends 2a, 2b of the pedicle screws 1 a, 1 b, i.e. upon a stronger bending of the pins 3a, 3b, the pins 3a, 3b abut against the internal surface of the cavities 5a, 5b over a greater part of their lengths.

Because the pins 3a, 3b, upon being bent further, abut against the internal surface of the cavities 5a, 5b over an increasingly greater part of their length, the length of the part of the pin 3a, 3b that can bend still further becomes increasingly shorter, so that the force which is required to effect such further bending increases progressively. A suitable internal surface of the cavity 5, in a sectional plane coinciding with the longitudinal center- plane of the pin 3a, 3b, can follow the contour of, for instance, a segment of a circle, a segment of a parabola or a like continuous contour.

It is clear that the contour of the internal surface need not be rotation-symmetrical with respect to the longitudinal axis of the pin 3. By choosing a non-rotation symmetrical contour, it can for instance be provided that the increase of the counteractive force upon a decrease of the distance between the free ends 2a, 2b of the pedicle screws 1 a, 1 b has a different curve than in the case where the distance is increased. Accordingly, it can thus be provided that more force is required for extension than for flexion.

The pin 3 can be secured in the cavity 5 by means of a securing bolt (not shown) extending transversely to the longitudinal axis of the pin 3. The pin 3 can also be connected with the free end 2 of the pedicle screw 1 by means of a press fit. Of course, other securing methods are possible as well.

In the exemplary embodiment shown, the pins 3 and the rigid connecting member 4 are constructed in one piece. The constriction 6 at the transition between the rigid connecting member 4 and the pins 3 creates the possibility of pivotal movement of the pins 3a, 3b relative to the rigid connecting member 4. It is clear that this possibility of pivotal movement can also be created in a different manner. Thus, the pins 3a, 3b may at the second end thereof be provided with a spherical body and the rigid connecting member 4 may be provided with cup-shaped cavities in which the spherical bodies are receivable. When placing the rigid connecting member 4, the spherical bodies are received in the cup-shaped cavities and thus form a ball-and-socket joint.

By manufacturing the pins 3 from memory metal, in particular from a TiNi alloy, a relatively short pin 3 yet enables a considerable elastic deformation of the pin and hence a relatively large displacement of the second end of the pins 3a, 3b through bending of the pins 3a, 3b. This is possible in that the memory metal features an elongation in the elastic range of 8%. For this application, no use is made of the mnemonic properties of the material.

In order to implant the device, first the pedicle screws 1 a, 1 are fitted at the appropriate points in two adjacent vertebrae V1, V2 (see Figs. 1, 2). Then the vertebral column is brought into the equilibrium or neutral position and the distance between the free ends 2a, 2b of the pedicle screws is accurately determined. Then a coupling element comprising the rigid connecting member 4 and the two pins 3a, 3b of the proper length is placed in the pedicle screws and secured, for instance by means of securing bolts or by tapping it home into a press fit.

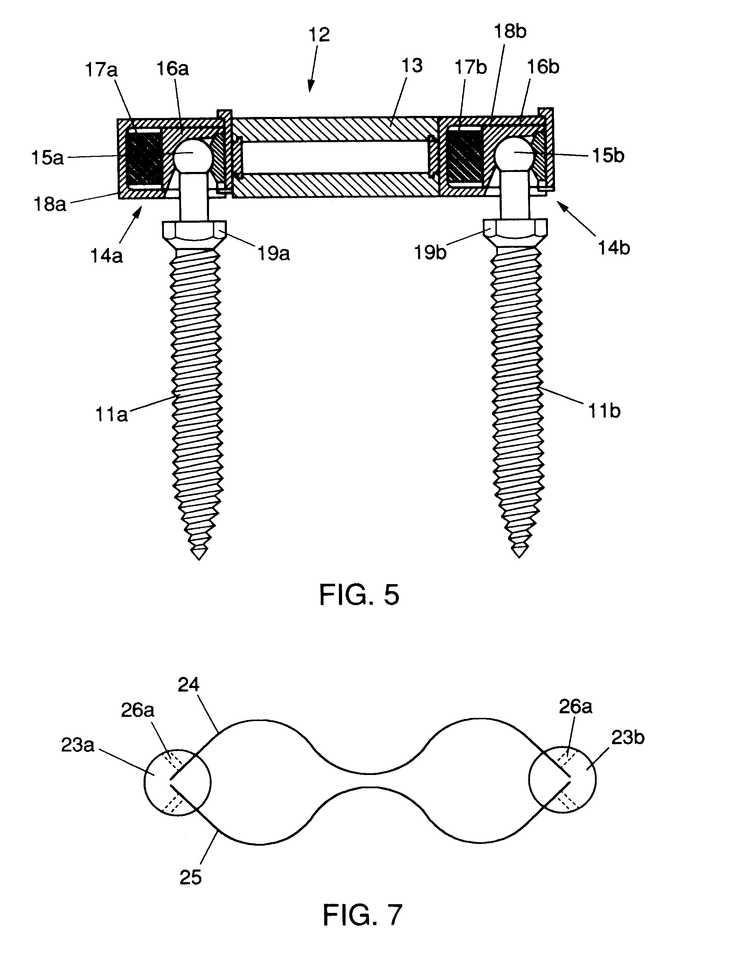

Fig. 5 shows an alternative embodiment of a device according to the invention, which comprises two vertebra engaging elements in the form of pedicle screws 11 a, 11 and a coupling element 12. The coupling element 12 comprises a rigid connecting member 13 and two ball-and-socket joints 14a, 14b, each comprising a ball 15a, 15b and a socket 16a, 16b. In mounted condition, each ball 15 is rigidly connected with the associated pedicle screw 11. Both sockets 16 are slidably connected with the rigid connecting member 13 and in a neutral position are urged in an end position by biassed springing means 17a, 17b. Fig. 5 shows the device in the neutral position. In the exemplary embodiment shown, the springing means 17a, 17b are formed by blocks 17a, 17b manufactured from rubber or plastic and accommodated in a chamber 18a, 18b. A block 17 accommodated in the chamber 18 can initially deform freely, until the block contacts the walls of the chamber 18 and the deformation is influenced. Because of this influence, the force required for further deformation of the rubber block 17 increases progressively.

In the exemplary embodiment shown in Fig. 5 the two sockets 16a, 16b are each positioned relative to the springing means 17a, 17b in such a manner that upon an increase of the distance between the free ends 15a, 15b of the pedicle screws 11 a, 11 one socket 16b is urged into the associated end position and the other socket 16a moves from the end position against the spring pressure of the springing means 17a associated with that socket 16a. Conversely, upon a decrease of the distance between the free ends 15a, 15b of the pedicle screws 11 a, 11 b, the above-mentioned other socket 16a is urged into the end position while the above-mentioned one socket 16b moves from the end position against the spring pressure of the springing means 16b associated with the one socket16b.

Optionally, the elasticity of one rubber block 17a may be chosen differently from that of the other rubber block 17b. Thus it can be provided that a greater force is required for moving the free ends 15a, 15b of the pedicle screws 11 a, 11b towards each other than for moving them apart.

In this embodiment, the pedicle screws 11 a, 11 b are each provided with a conical nut 19a, 19b, formed thereon, which facilitates tightening the pedicle screws 11 a, 11 and which also serves as a stop. In the embodiment of Figs. 3 and 4, this conical stop is integrated into the free end 2a, 2b of the pedicle screw 1 a, 1 b.

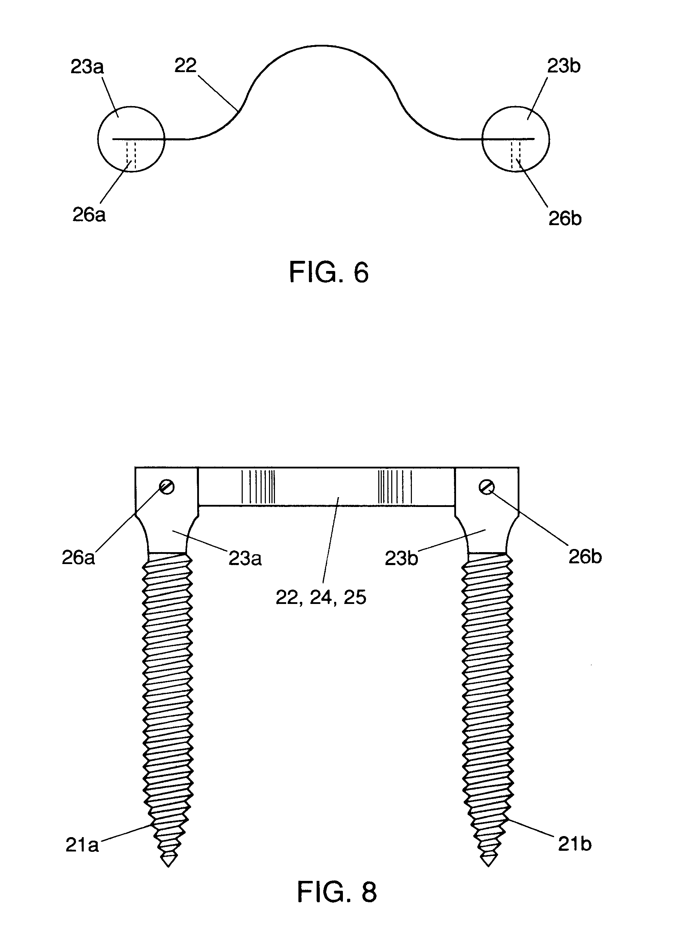

In a third embodiment, which is shown in Figs. 6 and 8, the coupling element comprises a leaf spring 22 which is provided with a curvature. The leaf spring 22 is clamped in a slot in the free ends 23a, 23b of the vertebra engaging elements, which in this exemplary embodiment are designed as pedicle screws 21 a, 21 b. In the exemplary embodiment shown, the clamping is effected by means of locking screws 26. It will be clear that a press fit, soldering, welding or other jointing techniques also qualify for this purpose. Upon an increase of the distance between the free ends 23a, 23b of the pedicle screws 21a, 21 b, the leaf spring 22 exerts on the pedicle screws a force counteracting the distance increase. According as the distance between the free ends 23a, 23b of the pedicle screws 21 a, 21 b increases, the curve in the leaf spring 22 is straightened, so that the spring arm becomes smaller and the force required to further extend the leaf spring increases progressively.

In order to effect the progressive increase of the counteractive force also in the case where the free ends 23a, 23b of the pedicle screws 21 a, 21 b move towards each other, in the fourth embodiment, shown in Fig. 7, the coupling element is made up of two leaf springs 24, 25. The curvatures of the two leaf springs 24, 25 are of such design that upon a decrease of the distance between the free ends 23a, 23b of the pedicle screws 21 a, 21 b the leaf springs 24, 25 butt against each other. Owing to the fact that the leaf springs 24, 25 will butt against each other upon a particular decrease of the distance between the free ends 23a, 23b of the pedicle screws 21 a, 21 b, a much greater counteractive force will have to be overcome for any further reduction of the distance. Through the interaction between the two leaf springs 24, 25 the spring characteristic thereof will change markedly, in particular because the leaf springs 24, 25 must bend over a shorter part of the length thereof. This change of the spring characteristic results in a progressive increase of the counteractive force upon a decrease of the distance between the free ends 23a, 23b of the pedicle screws 21 a, 21b. It will be clear that the invention is not limited to the exemplary embodiments described but that various modifications are possible within the purview of the invention.

## Claims

1. A device for implantation for the purpose of limiting movements between two vertebrae (V1, V2), comprising at least two vertebra engaging elements (1a, 1 b; 11 a, 11 b) each having a free end (2a, 2b; 15a, 15b), and an elastic coupling element (3a, 3b, 4; 12) which is connected with the free ends (2a, 2b; 15a, 15b) of the vertebra engaging elements (1a, 1 b; 11 a, 11 b), the elastic coupling element (3a, 3b, 4; 12) being designed in such a manner that upon an increase of the distance between the free ends (2a, 2b; 15a, 15b) of the vertebra engaging elements (1 a, 1 b; 11 a, 11 b) starting from a neutral position the coupling element (3a, 3b, 4; 12) generates a force counteracting the increase and upon a decrease of the distance between the free ends (2a, 2b; 15a, 15b) of the vertebra engaging elements (1a, 1b; 11 a, 11 b) starting from the neutral position the coupling element (3a, 3b, 4; 12) generates a force counteracting the decrease.

2. A device according to claim 1, characterized in that the force counteracting the increase or decrease of the distance between the free ends (2a, 2b; 15a, 15b) of the vertebra engaging elements (1 a, 1 b; 11 a, 11 b) increases progressively with the extent of increase or decrease.

3. A device according to claim 2, characterized in that the counteractive force increases exponentially with the extent of increase or decrease.

4. A device according to claim 2 or 3, characterized in that the counteractive force arising upon a particular increase of the distance between the free ends (2a, 2b; 15a, 15b) of the vertebra engaging elements (1 a, 1 b; 11 a, 11 b) is smaller than the counteractive force arising upon an equally large decrease of that distance.

5. A device according to any one of claims 1-4, characterized in that the coupling element (3a, 3b, 4) comprises two flexible pins (3a, 3b) which are each rigidly connected by one end with the free end (2a, 2b) of a vertebra engaging element (1 a, 1b) and are each connected at the other end with a rigid connecting member (4), each vertebra engaging element (1 a, 1b) at the free end (2a, 2b) thereof comprising an internal cavity (5) in which the pin (3a, 3b) extends at least partly, the internal surface of the cavity (5) serving to support the pin (3a, 3b) during the bending thereof.

6. A device according to claim 5, characterized in that the internal surface of the cavity (5) in the vertebra engaging element (1 a, 1 b) is designed in such a manner that upon a greater extent of increase or decrease of the distance between the free ends (2a, 2b) of the vertebra engaging elements (1 a, 1 b), i.e. upon stronger bending of the pins (3a, 3b), the pins (3a, 3b) abut against the internal surface over a greater part of their length.

7. A device according to claim 5 or 6, characterized in that the flexible pin (3a, 3b) is manufactured from memory metal, such as, for instance, a TiNi alloy.

8. A device according to any one of claims 5-7, characterized in that the rigid connecting member (4) is pivotally connected to the free ends of the two flexible pins (3a, 3b).

9. A device according to any one of claims 5-8, characterized in that the pins are designed as a bundle of substantially parallel wire-shaped elements.

10. A device according to any one of claims 1-4, characterized in that the coupling element (12) comprises a rigid connecting member (13) and two ball-and-socket joints (14a, 14b) each comprising a ball (15a, 15b) and a socket (16a, 16b), each ball (15a, 15b) in mounted condition being rigidly connected with an associated vertebra engaging element (11 a, 11 b), while each socket (16a, 16b) is slidably connected with the rigid connecting member (13) and in a neutral position is urged into an end position by biassed springing means (17a, 17b).

11. A device according to claim 10, characterized in that the springing means (17a, 17b) are formed by blocks (17a, 17b) made of rubber or plastic and accommodated in a chamber (18a, 18b) of the rigid connecting member (13).

12. A device according to claim 10 or 11, characterized in that the two sockets (16a, 16b) are each positioned relative to the springing means (17a, 17b) in such a manner that upon an increase of the distance between the free ends (15a, 15b) of the vertebra engaging elements (11 a, 11 b) one socket (16b) is urged into the associated end position and the other socket (16a) moves from the end position against the spring pressure of the springing means (17a) associated with that socket (16a), while upon a decrease of the distance between the free ends (15a, 15b) of the vertebra engaging elements (11 a, 11 b) said other socket (16a) is urged into the end position and said one socket (16b) moves from the end position against the spring pressure of the springing means (17b) associated with said one socket (16b).

13. A device according to any one of claims 1-4, characterized in that the coupling element comprises at least one spring element, manufactured from springing wire or sheet material, which is provided with a curvature, the or each spring element being connected at free ends thereof with the free ends of the vertebra engaging elements.

14. 14. A device according to claim 13, characterized in that the coupling element comprises two spring elements, the curvatures of the two elements being so designed that upon a decrease of the distance between the free ends of the vertebra engaging elements the spring elements butt against each other.

15. 15. A device according to any one of claims 13 or 14, characterized in that the spring elements are designed as leaf springs.
